# FASCICULE DE BREVET EUROPEEN

(11) **EP 1 002 536 B1**
(45) Date de publication et mention de la délivrance du brevet: **30.06.2004**
(21) Numéro de dépôt: 99402771.2
(22) Date de dépôt: 08.11.1999
(51) Int. Cl.: A61K 31/505, A61K 7/48

(54) **2-Amino, 4-alkylamino pyrimidine 3-oxydes comme apaisants dermatologiques**
2-Amino, 4-alkylamino pyrimidine 3-oxyde als lindernde Mittel auf der Haut
2-Amino, 4-alkylamino pyrimidine 3-oxydes as soothing agents for the skin

(30) Priorité: 12.11.1998 FR 9814211
(43) Date de publication de la demande: 24.05.2000
(73) Titulaire: L'OREAL, 75008 Paris (FR)
(72) Inventeur: Mahe, Yann, 91390 Morsang-sur-Orge (FR); Michelet, Jean-François, 94000 Creteil (FR); Breton, Lionel, 78000 Versailles (FR)
(74) Mandataire: Galup, Cédric Olivier Nicolas

(56) Documents cités:
- EP-A- 0 356 271
- EP-A- 0 522 964
- EP-A- 0 736 300
- MAHE, YANN ET AL: "A minoxidil-related compound lacking a C6 substitution still exhibits strong anti-lysyl hydroxylase activity in vitro" SKIN PHARMACOL. (1996), 9(3), 177-183 CODEN: SKPHEU;ISSN: 1011-0283, XP002106038

## Description

La présente invention a pour objet l'utilisation d'au moins un composé de la famille des 2-amino, 4-alkylamino pyrimidine 3-oxydes, dans ou pour la préparation d'une composition apaisante. L'invention a également pour objet un procédé de traitement cosmétique apaisant.

On sait que l'être humain subit des phénomènes de types agressifs qui se caractérisent par un sentiment d'inconfort cutané, des tiraillements, des démangeaisons, la sensation de chaleur cutanée, ou des rougeurs.

On recherche depuis de nombreuses années des substances permettant de lutter contre ces types d'agression.

Même si des solutions ont déjà été proposées, il demeure constamment intéressant de disposer de nouveaux produits à activité apaisante, notamment pour des perturbations cutanées mineures, telles que, par exemple, les peaux sensibles, l'inconfort cutané, les tiraillements cutanés, les démangeaisons cutanées, le gonflement cutané, la rougeur cutanée, la sensation de chaleur cutanée.

EP0736300 divulgue des dérivés de pyrimidine substitués en 6 et avec un groupement alkyle dans la position 4 (2-amino-4-propylamino-6-piperidinopyrimidine 3-oxyde et le 2-amino-4-propylamino-6-dimethylaminopyridine 3-oxyde) et leur utilisation pour le traitement d'affections dermatologiques, telles que l'eczéma ou la psoriasis.

Le but de la présente invention est donc de pouvoir disposer d'un produit nouveau présentant ainsi une activité apaisante tout en ne présentant pas d'effets secondaires notables.

Ce but et d'autres sont atteints par la présente invention qui a pour objet l'utilisation dans ou pour la préparation une composition apaisante, dans un milieu physiologiquement acceptable, d'au moins un composé de la famille des 2-amino, 4-alkylamino pyrimidine 3-oxydes.

Par milieu physiologiquement acceptable, on comprend un milieu compatible avec la peau, les muqueuses, les ongles, les cheveux.

Les composés de la famille des 2-amino, 4-alkylamino pyrimidine 3-oxydes, utilisables selon l'invention répondent à la formule générale (1) : 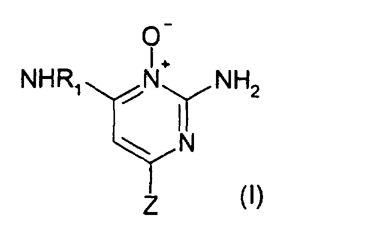 dans laquelle
R₁ représente un groupement alkyle ayant de 1 à 20 atomes de carbone,
et Z représente soit un atome d'hydrogène, ainsi que ses formes acylées ou ses sels d'addition d'acides.

Par radical alkyle, on entend selon l'invention un radical acyclique, linéaire ou ramifié, provenant de l'enlèvement d'un atome d'hydrogène dans la molécule d'un hydrocarbure, comme par exemple un radical méthyle, éthyle, propyle, butyle, pentyle, hexyle, heptyle, octyle, nonyle, décyle, undécyle, dodécyle, tridécyle, tétradécyle, pentadécyle, hexadécyle, heptadécyle, octodécyle, nonadécyle, eicosadécyle.

Selon une forme de réalisation préférentielle de l'invention, R₁ peut être un groupement alkyle ayant de 6 à 12 atomes de carbone, comme par exemple un radical hexyle, heptyle, octyle, nonyle, décyle, undécyle, dodécyle.

Préférentiellement, le composé utilisable selon l'invention est choisi parmi le
2-amino, 4-méthylamino pyrimidine 3-oxyde,
2-amino, 4-éthylamino pyrimidine 3-oxyde,
2-amino, 4-propylylamino pyrimidine 3-oxyde,
2-amino, 4-butylamino pyrimidine 3-oxyde,
2-amino, 4-pentylamino pyrimidine 3-oxyde,
2-amino, 4-hexylamino pyrimidine 3-oxyde,
2-amino, 4-heptylamino pyrimidine 3-oxyde,
2-amino, 4-octylamino pyrimidine 3-oxyde,
2-amino, 4-nonylamino pyrimidine 3-oxyde,
2-amino, 4-décylamino pyrimidine 3-oxyde,
2-amino, 4-undécylamino pyrimidine 3-oxyde,
2-amino, 4-dodécylamino pyrimidine 3-oxyde,
2-amino, 4-tridécylamino pyrimidine 3-oxyde,
2-amino, 4-tétradécylamino pyrimidine 3-oxyde,
2-amino, 4-pentadécylamino pyrimidine 3-oxyde,
2-amino, 4-hexadécylamino pyrimidine 3-oxyde,
2-amino, 4-heptadécylamino pyrimidine 3-oxyde,
2-amino, 4-octadécylamino pyrimidine 3-oxyde,
2-amino, 4-nonadécylamino pyrimidine 3-oxyde, et leurs isomères ramifiés.

Préférentiellement selon l'invention, on utilise un composé choisi parmi le :
2-amino, 4-hexylamino pyrimidine 3-oxyde,
2-amino, 4-octylamino pyrimidine 3-oxyde,
2-amino, 4-dodécylamino pyrimidine 3-oxyde,

Et encore plus préférentiellement, on utilise un composé choisi parmi le
2-amino, 4-dodécylamino pyrimidine 3-oxyde,

Bien entendu selon l'invention, les composés de la famille des 2-amino, 4-alkylamino pyrimidine 3-oxydes peuvent être utilisés seuls ou en mélange.

Ainsi, selon un aspect particulier, l'invention a pour objet l'utilisation dans ou pour la préparation d'une composition, dans un milieu physiologiquement acceptable, d'au moins composé de la famille des 2-amino, 4-alkylamino pyrimidine 3-oxydes répondant à la formule générale (I), le composé ou la composition étant destinés à lutter contre les peaux sensibles, les perturbations cutanées telles que l'inconfort cutané, les tiraillements cutanés, les démangeaisons cutanées, le gonflement cutané, la rougeur cutanée, la sensation de chaleur cutanée.

La composition de l'invention dans laquelle au moins un composé de la famille des 2-amino, 4-alkylamino pyrimidine 3-oxydes peut être une composition cosmétique.

La quantité de composés de la famille des 2-amino, 4-alkylamino pyrimidine 3-oxydes utilisable selon l'invention dépend bien évidemment de l'effet recherché et doit être en une quantité efficace pour combattre les perturbations cutanées visées.

A titre d'exemple la quantité de composés de la famille des 2-amino, 4-alkylamino pyrimidine 3-oxydes utilisable selon l'invention peut aller par exemple de 0,01% à 20% et de préférence de 0,05% à 10% du poids total de la composition.

Cette composition peut être administrée par toute voie envisageable. De préférence par application topique sur la peau.

Le milieu physiologiquement acceptable dans lequel le composé de la famille des 2-amino, 4-alkylamino pyrimidine 3-oxydes est utilisé selon l'invention peut être anhydre ou aqueux. On entend par milieu anhydre, un milieu solvant contenant moins de 1% d'eau. Ce milieu peut être constitué d'un solvant ou d'un mélange de solvants choisi plus particulièrement parmi les alcools inférieurs en C₂-C₄ comme l'alcool éthylique, les alkylèneglycols comme le propylèneglycol, et les alkyléthers d'alkylèneglycols ou de dialkylèneglycols, dont les radicaux alkyle ou alkylène contiennent de 1 à 4 atomes de carbone. On entend par milieu aqueux, un milieu constitué par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.

Il est possible que le milieu physiologiquement acceptable contienne d'autres adjuvants habituellement utilisés dans le domaine cosmétique, tels que des agents tensioactifs, des agents épaississants ou gélifiants, des agents cosmétiques, des agents conservateurs, des agents alcalinisants ou acidifiants bien connus dans l'état de la technique, et en quantités suffisantes pour obtenir la forme de présentation désirée, notamment de lotion plus ou moins épaissie, de gel, d'émulsion, ou de crème. L'utilisation peut éventuellement se faire sous une forme pressurisée en aérosol ou vaporisée à partir d'un flacon pompe.

Selon l'invention, la composition peut associer au moins un composé de formule
(I) à d'autres agents actifs. Parmi ces agents actifs, on peut citer à titre d'exemple :
   - d'autres agents apaisants comme des peptides comme par exemple le tripeptide Lysine-Proline-Valine ;
   - les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
   - les agents anti-radicaux libres, tels que l'α-tocophérol ou ses esters, les superoxyde dismutases, certains chélatants de métaux ou l'acide ascorbique et ses esters ;
   - les extraits d'origine végétale, marine ou bactérienne.

A la liste ci-dessus, d'autres composés peuvent également être rajoutés, à savoir par exemple des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des caroténoides, les acides eicosatétraénoïque et eicosatriénoïque ou leurs esters et amides, la vitamine D et ses dérivés.

Le composé de la famille des 2-amino, 4-alkylamino pyrimidine 3-oxydes, ou la composition cosmétique le comprenant, est à appliquer sur les zones de la peau et/ou du cuir chevelu d'un individu qui le nécessitent, éventuellement à laisser en contact plusieurs heures et éventuellement à rincer.

Ainsi, la présente invention à également pour objet un procédé de traitement cosmétique apaisant destiné à lutter contre les peaux sensibles, les perturbations cutanées telles que l'inconfort cutané, les tiraillements cutanés, les démangeaisons cutanées, le gonflement cutané, la rougeur cutanée, la sensation de chaleur cutanée, caractérisé par le fait qu'il consiste à appliquer sur la peau et/ou le cuir chevelu d'un individu une composition cosmétique comprenant au moins un composé de la famille des 2-amino, 4-alkylamino pyrimidine 3-oxydes, à laisser celle-ci en contact avec la peau et/ou le cuir chevelu, et éventuellement à rincer.

Le procédé de traitement cosmétique permet ainsi d'améliorer l'esthétique et le confort de la peau et/ou du cuir chevelu et d'améliorer le confort d'un individu en traitant les effets cutanés d'une agression.

On va maintenant donner à titre d'illustration des exemples qui ne sauraient limiter en aucune façon la portée de l'invention.

Exemple 1 : Exemples de compositions. Ces compositions ont été obtenues par simple mélange des différents composants.

### Lotion :

- 2-amino, 4-dodécylamino pyrimidine 3-oxyde 1,500 %
- Propylène glycol 10,000 %
- Alcool isopropylique qsp 100 %

On applique 1 ml de cette lotion sur le cuir chevelu, à la fréquence de une à deux fois par jour.

### Lotion :

- 2-amino, 4-dodécylamino pyrimidine 3-oxyde 1,000 %
- Propylène glycol 30,000 %
- Alcool éthylique 40.500 %
- Eau qsp 100 %

On applique cette lotion une à deux fois par jour, à raison d'1 ml par application.

### Lotion épaissie :

- 2-amino, 4-octylamino pyrimidine 3-oxyde 2,000 %
- Kawaïne 2,000 %
- Hydroxypropylcellulose vendue par la société Hercules sous la dénomination Klucel G① 3,500 %
- Alcool éthylique qsp 100 %

On applique cette lotion épaissie une à deux fois par jour, .

### Lotion :

- Chimexane NL① 0,475 %
- Cholestérol 0,475 %
- Stéaroylglutamate monosodique 0,050 %
- 2-amino, 4-dodécylamino pyrimidine 3-oxyde 0,500 %
- Conservateurs qs
- Colorants qs
- Parfum qs
- Eau déminéralisée qsp 100 %

On applique cette lotion une à deux fois par jour, à raison d'1 ml par application.

### lotion :

- 2-amino, 4-hexylamino pyrimidine 3-oxyde 0,100 %
- Monométhyléther de propylèneglycol vendu sous la dénomination Dowanol PM① par la société Dow Chemical 20,000 %
- Hydroxypropylcellulose vendue par la société Herculès sous la dénomination Klucel G① 3,000 %
- Alcool éthylique 40,000 %
- Eau qsp 100 %

On applique cette lotion épaissie à raison d'1 ml par application.

### Crème de jour :

- 2-amino, 4-dodécylamino pyrimidine 3-oxyde 1.000 %
- stéarate de sucrose 4,000 %
- alcool stéarylique 2,000 %
- cyclohexasiloxane 9,000 %
- huile minérale 4,000 %
- glycérine 5,000 %
- gomme de xanthane 0.300 %
- carbomer 0.500 %
- conservateurs 0.300 %
- parfum 0.300 %
- eau qsp100 %

### Fluide de soin :

- 2-amino, 4-dodécylamino pyrimidine 3-oxyde 1,000 %
- Alcool stéarylique 0.400 %
- Stéarate de sorbitan 1.500 %
- Glycérine 5,000 %
- Gomme de xanthane 0.200 %
- Carbomer 0.100 %
- Cyclohexasiloxane 7,000 %
- Conservateurs 0.300 %
- Parfum 0.200 %
- Eau qsp100 %

### Lotion :

- 2-amino, 4-dodécylamino pyrimidine 3-oxyde 0,500 %
- Propylenglycole 2,000 %
- Extrait de fleurs de bleuet 0.100 %
- Conservateurs 0.100 %
- PEG 60 hydrogenated castor oil 0.400 %
- Parfum 0.100 %
- Eau qsp100 %

## Revendications

1. Utilisation dans un milieu physiologiquement acceptable, d'au moins un composé de la famille des 2-amino, 4-alkylamino pyrimidine 3-oxydes de formule générale (I), 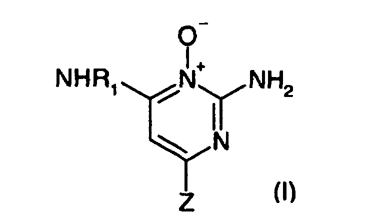 dans laquelle
R₁ représente un groupement alkyle ayant de 1 à 20 atomes de carbone,
et Z représente un atome d'hydrogène, ,
ainsi que ses formes acylées ou ses sels d'addition d'acides,
dans ou pour la préparation d'une composition ayant un effet apaisant.

2. Utilisation selon la revendication 1, carcatérisée en ce que le composé ou la composition est destiné au traitement des perturbations cutanées telles que les peaux sensibles, l'inconfort cutané, les tiraillements cutanés, les démangeaisons cutanées, le gonflement cutané, la rougeur cutanée, la sensation de chaleur cutanée.

3. Utilisation selon l'une quelconque des revendications précédentes, **caractérisée par le fait que** R₁ est un groupement alkyle ayant de 6 à 12 atomes de carbone.

4. Utilisation selon l'une quelconque des revendications précédentes, **caractérisée par le fait que** le composé de formule (I) est choisi parmi le :
2-amino, 4-méthyiamino pyrimidine 3-oxyde,
2-amino, 4-éthylamino pyrimidine 3-oxyde,
2-amino, 4-propylylamino pyrimidine 3-oxyde,
2-amino, 4-butylamino pyrimidine 3-oxyde,
2-amino, 4-pentylamino pyrimidine 3-oxyde,
2-amino, 4-hexylamino pyrimidine 3-oxyde,
2-amino, 4-heptylamino pyrimidine 3-oxyde,
2-amino, 4-octylamino pyrimidine 3-oxyde,
2-amino, 4-nonylamino pyrimidine 3-oxyde,
2-amino, 4-décylamino pyrimidine 3-oxyde,
2-amino, 4-undécylamino pyrimidine 3-oxyde,
2-amino, 4-dodécylamino pyrimidine 3-oxyde,
2-amino, 4-tridécylamino pyrimidine 3-oxyde,
2-amino, 4-tétradécylamino pyrimidine 3-oxyde,
2-amino, 4-pentadécylamino pyrimidine 3-oxyde,
2-amino, 4-hexadécylamino pyrimidine 3-oxyde,
2-amino, 4-heptadécylamino pyrimidine 3-oxyde,
2-amino, 4-octadécylamino pyrimidine 3-oxyde,
2-amino, 4-nonadécylamino pyrimidine 3-oxyde,
2-amino, 4-eicosadécylamino pyrimidine 3-oxyde,
et leurs isomères ramifiés.

5. Utilisation selon la revendication 4, **caractérisée par le fait que** le composé est choisi parmi le :
2-amino, 4-hexylamino pyrimidine 3-oxyde,
2-amino, 4-octylamino pyrimidine 3-oxyde,
2-amino, 4-dodécylamino pyrimidine 3-oxyde.

6. Utilisation selon l'une quelconque des revendications 4 ou 5, **caractérisée par le fait que** le composé est le :
2-amino, 4-dodécylamino pyrimidine 3-oxyde.

7. Utilisation selon l'une quelconque des revendications précédentes, **caractérisée par le fait que** la quantité de composés de la famille des 2-amino, 4-alkylamino pyrimidine 3-oxydes utilisable selon l'invention est comprise entre 0,01% à 20% du poids total de la composition.

8. Utilisation selon la revendication précédente, **caractérisée par le fait que** la quantité de composés de la famille des 2-amino, 4-alkylamino pyrimidine 3-oxydes utilisabie selon l'invention est comprise entre 0,05% à 10% du poids total de la composition.

9. Procédé de traitement cosmétique non thérapeutique apaisant de la peau et/ou du cuir chevelu d'un individu, **caractérisé par le fait qu'**il consiste à appliquer sur la peau et/ou le cuir chevelu, une composition cosmétique comprenant au moins un composé de la famille des 2-amino, 4-alkylamino pyrimidine 3-oxydes tel que décrit dans l'une quelconque des revendications 1 à 8, à laisser celle-ci en contact avec la peau et/ou le cuir chevelu, et éventuellement à rincer.

## Patentansprüche

1. Verwendung mindestens einer Verbindung aus der Gruppe der 2-Amino-4-alkylamino-pyrimidin-3-oxide der folgenden allgemeinen Formel (I) in einem physiologisch akzeptablen Medium: 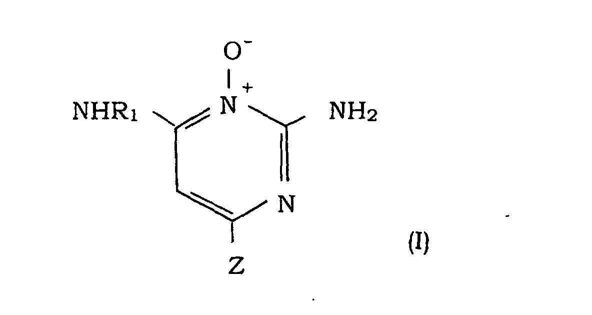 worin bedeuten:
R₁ eine Alkylgruppe mit 1 bis 20 Kohlenstoffatomen, und
Z ein Wasserstoffatom,
sowie die acylierten Formen dieser Verbindungen oder ihre Additionssalze mit Säuren, in einer Zusammensetzung oder zur Herstellung einer Zusammensetzung, die eine beruhigende Wirkung besitzt.

2. Verwendung nach Anspruch 1, **dadurch gekennzeichnet, dass** die Verbindung oder die Zusammensetzung dazu vorgesehen sind, Hautstörungen zu bekämpfen, wie beispielsweise empfindliche Haut, unangenehmes Hautgefühl, Spannen der Haut, Juckreiz an der Haut, gequollene Haut, Hautrötungen und Wärmegefühl auf der Haut.

3. Verwendung nach einem der vorhergehenden Ansprüche, **dadurch gekennzeichnet, dass** R₁ eine Alkylgruppe mit 6 bis 12 Kohlenstoffatomen ist.

4. Verwendung nach einem der vorhergehenden Ansprüche, **dadurch gekennzeichnet, dass** die Verbindung der Formel (I) unter den folgenden Verbindungen ausgewählt ist:
2-Amino-4-methylamino-pyrimidin-3-oxid,
2-Amino-4-ethylamino-pyrimidin-3-oxid,
2-Amino-4-propylamino-pyrimidin-3-oxid,
2-Amino-4-butylamino-pyrimidin-3-oxid,
2-Amino-4-pentylamino-pyrimidin-3-oxid,
2-Amino-4-hexylamino-pyrimidin-3-oxid,
2-Amino-4-heptylamino-pyrimidin-3-oxid,
2-Amino-4-octylamino-pyrimidin-3-oxid,
2-Amino-4-nonylamino-pyrimidin-3-oxid,
2-Amino-4-decylamino-pyrimidin-3-oxid,
2-Amino-4-undecylamino-pyrimidin-3-oxid,
2-Amino-4-dodecylamino-pyrimidin-3-oxid,
2-Amino-4-tridecylamino-pyrimidin-3-oxid,
2-Amino-4-tetradecylamino-pyrimidin-3-oxid,
2-Amino-4-pentadecylamino-pyrimidin-3-oxid,
2-Amino-4-hexadecylamino-pyrimidin-3-oxid,
2-Amino-4-heptadecylamino-pyrimidin-3-oxid,
2-Amino-4-octadecylamino-pyrimidin-3-oxid,
2-Amino-4-nonadecylamino-pyrimidin-3-oxid,
2-Amino-4-eicosadecylamino-pyrimidin-3-oxid
und deren verzweigten Isomeren.

5. Verwendung nach Anspruch 4, **dadurch gekennzeichnet, dass** die Verbindung ausgewählt ist unter:
2-Amino-4-hexylamino-pyrimidin-3-oxid,
2-Amino-4-octylamino-pyrimidin-3-oxid,
2-Amino-4-dodecylamino-pyrimidin-3-oxid.

6. Verwendung nach einem der Ansprüche 4 oder 5, **dadurch gekennzeichnet, dass** es sich bei der Verbindung um das 2-Amino-4-dodecylamino-pyrimidin-3-oxid handelt.

7. Verwendung nach einem der vorhergehenden Ansprüche, **dadurch gekennzeichnet, dass** der erfindungsgemäß verwendbare Mengenanteil der Verbindungen aus der Gruppe der 2-Amino-4-alkylamino-pyrimidin-3-oxide im Bereich von 0,01 bis 20 % des Gesamtgewichts der Zusammensetzung liegt.

8. Verwendung nach dem vorhergehenden Anspruch, **dadurch gekennzeichnet, dass** der erfindungsgemäß verwendbare Mengeanteil der Verbindungen aus der Gruppe der 2-Amino-4-alkylaminopyrimidin-3-oxide im Bereich von 0,05 bis 10 % des Gesamtgewichts der Zusammensetzung liegt.

9. Nichttherapeutisches Verfahren zur kosmetischen beruhigenden Behandlung der Haut und/oder der Kopfhaut einer Person, **dadurch gekennzeichnet, dass** es darin besteht, auf die Haut und/oder die Kopfhaut eine kosmetische Zusammensetzung aufzutragen, die mindestens eine Verbindung der aus der Gruppe der 2-Amino-4-alkylamino-pyrimidin-3-oxide nach einem der Ansprüche 1 bis 8 enthält, diese mit der Haut und/oder mit der Kopfhaut in Kontakt zu belassen und gegebenenfalls zu spülen.

## Claims

1. Use, in a physiologically acceptable medium, of at least one compound of the 2-amino-4-alkylaminopyrimidine 3-oxide family of general formula (I) : 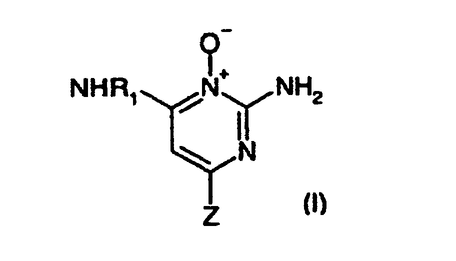 in which
R₁ represents an alkyl group containing from 1 to 20 carbon atoms,
and Z represents a hydrogen atom,
as well as the acyl forms thereof or the addition salts thereof with acids,
in or for the preparation of a composition having a soothing effect.

2. Use according to Claim 1, **characterized in that**, the compound or the composition is intended to treat skin complaints such as sensitive skin, skin discomfort, tautness of the skin, itchiness of the skin, swelling of the skin, blotchy red skin or a sensation of hotness on the skin.

3. Use according to either of the preceding claims, **characterized in that** R₁ is an alkyl group containing from 6 to 12 carbon atoms.

4. Use according to any one of the preceding claims, **characterized in that** the compound of formula (I) is chosen from:
2-amino-4-methylaminopyrimidine 3-oxide,
2-amino-4-ethylaminopyrimidine 3-oxide,
2-amino-4-propylaminopyrimidine 3-oxide,
2-amino-4-butylaminopyrimidine 3-oxide,
2-amino-4-pentylaminopyrimidine 3-oxide,
2-amino-4-hexylaminopyrimidine 3-oxide,
2-amino-4-heptylaminopyrimidine 3-oxide,
2-amino-4-octylaminopyrimidine 3-oxide,
2-amino-4-nonylaminopyrimidine 3-oxide,
2-amino-4-decylaminopyrimidine 3-oxide,
2-amino-4-undecylaminopyrimidine 3-oxide,
2-amino-4-dodecylaminopyrimidine 3-oxide,
2-amino-4-tridecylaminopyrimidine 3-oxide,
2-amino-4-tetradecylaminopyrimidine 3-oxide,
2-amino-4-pentadecylaminopyrimidine 3-oxide,
2-amino-4-hexadecylaminopyrimidine 3-oxide,
2-amino-4-heptadecylaminopyrimidine 3-oxide,
2-amino-4-octadecylaminopyrimidine 3-oxide,
2-amino-4-nonadecylaminopyrimidine 3-oxide,
2-amino-4-eicosadecylaminopyrimidine 3-oxide,
and the branched isomers thereof.

5. Use according to Claim 4, **characterized in that** the compound is chosen from:
2-amino-4-hexylaminopyrimidine 3-oxide,
2-amino-4-octylaminopyrimidine 3-oxide,
2-amino-4-dodecylaminopyrimidine 3-oxide.

6. Use according to either of Claims 4 and 5, **characterized in that** the compound is:
2-amino-4-dodecylaminopyrimidine 3-oxide.

7. Use according to any one of the preceding claims, **characterized in that** the amount of compounds of the 2-amino-4-alkylaminopyrimidine 3-oxide family which can be used according to the invention is between 0.01% and 20% of the total weight of the composition.

8. Use according to the preceding claim, **characterized in that** the amount of compounds of the 2-amino-4-alkylaminopyrimidine 3-oxide family which can be used according to the invention is between 0.05% and 10% of the total weight of the composition.

9. Soothing, non-therapeutic, cosmetic treatment process for the skin and/or the scalp of an individual, **characterized in that** it consists in applying a cosmetic composition comprising at least one compound of the 2-amino-4-alkylaminopyrimidine 3-oxide family as described in any one of Claims 1 to 8 to the skin and/or the scalp, in leaving this composition in contact with the skin and/or the scalp, and optionally in rinsing it off.
